# EUROPÄISCHE PATENTANMELDUNG

(11) **EP 0 773 210 A1**
(43) Veröffentlichungstag der Anmeldung: **14.05.1997**
(21) Anmeldenummer: 96116755.8
(22) Anmeldetag: 18.10.1996
(51) Int. Cl.: C07C 201/12, C07C 51/363, C07B 39/00

(54) **Verfahren zur Herstellung von fluorierten Aromaten**

(30) Priorität: 11.11.1995 DE 19542148
(71) Anmelder: HOECHST AKTIENGESELLSCHAFT, 65929 Frankfurt am Main (DE)
(72) Erfinder: Pasenok, Sergej, Dr., 65835 Liederbach (DE); Appel, Wolfgang, Dr., 65779 Kelkheim (DE)

(57) **Zusammenfassung**

2.1 Es ist bekannt, fluorierte aromatische Verbindungen der allgemeinen Formel I 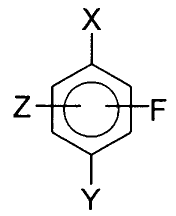 worin
   X, Y und Z die in der Beschreibung angegebene Bedeutung annehmen können, durch Umsetzung von aromatischen Verbindungen der allgemeinen Formel II 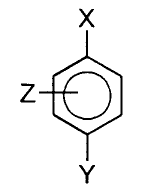 worinX, Y und Z die bei Formel I angegebene Bedeutung besitzen,
   mit Fluor in einem Reaktionsmedium herzustellen.
2.2 Erfindungsgemäß wird die Direktfluorierung in einem Reaktionsmedium enthaltend Polyfluoroalkansulfonsäuren der allgemeinen Formel III

   CFₙH₃₋ₙ(CFY)ₘ-SO₃H (III)

   worin
   m, n und Y die in der Beschreibung angegebene Bedeutung besitzen, durchgeführt.
   Hierdurch gelingt es besonders vorteilhaft ein Verfahren bereitzustellen, welches die bekannten Verfahren sowohl hinsichtlich der Selektivität, der Ausbeute als auch der Qualität der resultierenden Verfahrensprodukte in nicht ohne weiteres vorhersehbarer Weise verbessert.
2.3 Fluorierte aromatische Verbindungen der Formel I als Intermediate für die Wirkstoffsynthese.

## Beschreibung

Die Erfindung betrifft ein Verfahren zur Herstellung von fluorierten aromatischen Verbindungen der allgemeinen Formel I 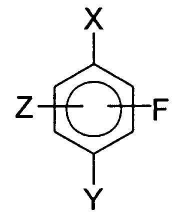 worin
X für COOH, COOR, CONH₂, CONR¹R², CF₃, CN, CHO, NO₂ oder NH₂,
Y für F, Cl, OCH₃, OCF₃, OCCl₃, p-NO₂-C₆H₄, p-NO₂-C₆H₄-O oder OH, und Z für H, F, Cl, CF₃, CCl₃ oder OCH₃ steht,
wobei R, R¹ und R² unabhängig voneinander gleich oder verschieden C₁-C₆-Alkyl, linear oder verzweigt, sein können und die Alkylreste R, R¹ und R² gegebenenfalls bis zu dreifach mit Halogen substituiert sein können,
durch Umsetzung von aromatischen Verbindungen der allgemeinen Formel II 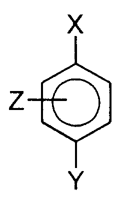 worin
X, Y und Z die bei Formel I angegebene Bedeutung besitzen, mit Fluor in einem Reaktionsmedium.

Die Substitution von aromatisch gebundenem Wasserstoff durch Fluor hat große Bedeutung für die Synthese von bioaktiven Substanzen respektive für die Herstellung von Vorstufen zu solchen Verbindungen.

Der Einsatz von molekularem Fluor für den gezielten selektiven Ersatz von einzelnen Wasserstoffatomen ist allerdings bislang auf wenige Fälle beschränkt. Dies findet seine Ursache u. a. darin, daß die Fluorierung von Aromaten im wesentlichen über einen radikalischen Mechanismus mit nur geringer oder vollständig fehlender Selektivität verläuft. Dabei kommt es durch Spaltung der organischen Moleküle und Rekombination der Fragmente vermehrt zur Bildung von Nebenprodukten.

Zum näheren Stand der Technik werden die Druckschriften
- D1 =: F.Cacace et al., J. Am. Chem. Soc. 102 (1980) 3511,
- D2 =: S. T. Purrington et al., J. Org. Chem. 56 (1991) 142,
- D3 =: EP-A-0 566 268,
- D4 =: M. van der Puy, Tetrahedron Lett. 28 (1987) 255, und
- D5 =: Chambers et al., J. Chem. Soc. Chem. Commun., 1995, Seite 17 genannt.

Zur Reduzierung oder Vermeidung des Nebenproduktanfalls wurden beispielsweise monosubstituierte Benzole bei -78°C in CCl₃F oder CH₃CN mit stark durch N₂ verdünntem molekularen Fluor umgesetzt (D1).

In D2 wurde die direkte Fluorierung aromatischer Substrate vom Typ ΦZ, worin Z für Cl, CHO, NO₂, OH, NHCH₃, OCH₃ oder CH₃ steht, mit und ohne Zusatz von BCl₃ oder AlCl₃ untersucht. Es wurde gefunden, daß sich durch einen Zusatz von BCl₃ sowohl der Reaktionsumsatz als auch die Ausbeute an para Isomer steigern lassen.

Ein Verfahren zur Herstellung von 3- und 5-trifluor-substituierten aromatischen Verbindungen ist Gegenstand der D3. Bei dem in der D3 geschilderten Verfahren werden als Edukte aromatische Verbindungen eingesetzt, die in 1-Stellung eine die Elektronendichte erniedrigende Gruppe und in 2- und 4-Position Elektronendonator-Gruppen als die Elektronendichte im aromatischen Ringsystem erhöhende Gruppen aufweisen.

Aus D4 ist es bekannt, daß die Direktfluorierung von in 4-Stellung substituierten Pyridinen bei tiefen Temperaturen zur Bildung von 2-Fluorderivaten mit Rohausbeuten von zwischen 25 und 60 % (wt/wt) führt.

Kürzlich zeigten die Autoren der D5, daß die Fluorierung aromatischer Verbindungen mit F₂ in stark polaren Lösungsmitteln wie beispielweise HCOOH und H₂SO₄ in verhältnismäßig hohen Ausbeuten zu fluorierten Verbindungen führt, wobei gleichzeitig die Selektivität der Umsetzung verbessert ist. Die besten Ergebnisse werden gemäß D5 bei der direkten Fluorierung von 1,4-disubstituierten Benzolderivaten der allgemeinen Formel IV 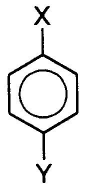 worin
X für COOH oder NO_{2,} und
Y für F, Cl oder OCH₃ steht,
unter Verwendung von konzentrierter H₂SO₄ als stark polarem Reaktionsmedium erzielt.

Diese Methode ist besonders für Aromaten mit Substitutionsmustern geeignet, die zu weiteren selektiven Substitutionen führen. Hierzu gehören vor allem disubstituierte Aromaten, die je einen aktivierenden und einen desaktivierenden Substituenten enthalten und dementsprechend in o/p- bzw. m-Position dirigieren.

Die Anwendung von Schwefelsäure als Medium für eine Direktfluorierung von Aromaten weist eine Reihe gravierender Nachteile auf.
i. Die Löslichkeit von aromatischen Verbindungen in konzentrierter Schwefelsäure ist im allgemeinen sehr gering. Hieraus ergibt sich die Notwendigkeit in relativ verdünnten Lösungen (etwa 3 - 10 Gew.-% (wt/wt)) zu arbeiten, was technische Nachteile mit sich bringt.
i. Die Begasung mit molekularem Fluor erfordert eine sehr hohe Feinheit der Gasbläschen und der Gasverteilung. Diese wird in der Regel durch sehr hohe Reaktoren und mehrstufige Rührer erreicht. Insbesondere das Reaktionsmedium Schwefelsäure mit seiner sehr hohen Oberflächenspannung und einer vergleichsweise sehr geringen Löslichkeit für Fluor erfordert diese aufwendigen apparativen Maßnahmen, um das elementare Fluor vollständig zu verbrauchen. Dies bedeutet de facto lange Reaktionszeiten, hohen Energieverbrauch und hohe Apparatekosten.
i. Die Verwendung von Schwefelsäure als Medium für die Direktfluorierung macht die Aufarbeitung schwierig. Im allgemeinen ist die Verdünnung des kompletten Reaktionsmediums mit Wasser nötig, um die Zielprodukte zu isolieren. Dies führt zu einer entsprechenden Abwasserbelastung.

Angesichts des hierin angegebenen und diskutierten Standes der Technik war es mithin Aufgabe der Erfindung, ein Verfahren der eingangs erwähnten Gattung anzugeben, das die Herstellung definierter Zielverbindungen in guter Ausbeute mit hoher Selektivität erlaubt. Das neue Verfahren soll für den großtechnischen Einsatz tauglich sein, möglichst wenig umweltbelastend und gleichzeitig mit relativ einfachen Mitteln kostengünstig zu realisieren sein. Dabei soll das Verfahren insbesondere von den oben erwähnten Nachteilen frei sein.

Gelöst werden diese sowie weitere nicht näher angegebene Aufgaben durch ein Verfahren der eingangs erwähnten Art mit dem Merkmal des kennzeichnenden Teils des Anspruches 1. Vorteilhafte Modifikationen des Verfahrens der Erfindung werden in den von Anspruch 1 abhängigen Unteransprüchen unter Schutz gestellt. Eine zur Erfindung gehörende Verwendung ist Gegenstand der Ansprüche 12 und 13.

Dadurch, daß die Direktfluorierung in einem Reaktionsmedium enthaltend Polyfluoroalkansulfonsäuren der allgemeinen Formel III

CFₙH₃₋ₙ(CFY)ₘ-SO₃H (III)

worin
m 0 oder eine positive ganze Zahl aus dem Bereich 1 bis 5, n eine positive ganze Zahl aus dem Bereich 1 bis 3 und Y F, Cl, H oder RFY' ist, wobei Y' F, Cl oder H ist und R C₁₋₃-Alkylen, linear oder verzweigt, ist, welches gegebenenfalls teilweise fluoriert oder auch perfluoriert sein kann, durchgeführt wird, gelingt es besonders vorteilhaft ein Verfahren bereitzustellen, welches die bekannten Verfahren sowohl hinsichtlich der Selektivität, der Ausbeute als auch der Qualität der resultierenden Verfahrensprodukte in nicht ohne weiteres vorhersehbarer Weise verbessert. Insbesondere weist das Verfahren der Erfindung folgende Vorteile auf:

Im Vergleich zu Schwefelsäure ist die Löslichkeit von Aromaten in Polyfluoralkansulfonsäuren wesentlich besser, was den Vorteil hat, daß man mit einer 10 - 25 Gew.-% (wt/wt) Lösung der aromatischen Substrate im Reaktionsmedium arbeiten kann.
I. Gleichzeitig ist die Löslichkeit von F₂ in Polyfluoralkansulfonsäuren der allgemeinen Formel III größer als in H₂SO₄, so daß das elementare Fluor ohne großen technischen Aufwand vollständig verbraucht und die Reaktionszeit und der Energieverbrauch reduziert werden.
i. Die Aufarbeitung des Reaktionsgemisches nach der Fluorierung kann durch destilative Auftrennung des Reaktionsgemisches erfolgen und ermöglicht es, die Polyfluoralkansulfonsäuren zu isolieren und zu recyclieren.

Als Verdünnungsmittel oder Lösungsmittel kommen erfindungsgemäß Polyfluoralkansulfonsäuren der allgemeinen Formel III in Frage. Diese Verbindungen sind zum überwiegenden Teil käuflich zu erwerben und damit verfügbar. Verbindungen der allgemeinen Formel III, welche nicht kommerziell erhältlich sind, lassen sich auf einfache Weise nach dem Fachmann geläufigen Verfahren synthetisieren. Die genannten Reaktionsmedien können als Verdünnungsmittel, Lösungsmittel oder Zusatz zu solchen Mitteln eingesetzt werden. Die erfindungsgemäßen Polyfluoroalkansulfonsäuren können in reiner Form oder im Gemisch von mehr als einer Verbindung eingesetzt werden.

Die verschiedenen zur allgemeinen Formel III gehörigen Verbindungen haben unterschiedliche Siedepunkte. Je nach Siedepunkt der aromatischen Edukte und Produkte und abhängig von der angestrebten Produktaufarbeitung kann eine für jeden einzelnen Fall maßgeschneiderte Verbindung der Formel III ausgewählt werden, sei es als Zusatz zu anderen Reaktionsmedien, sei es als Reaktionsmedium in reiner Form oder im Gemisch.

Zu bevorzugt erfindungsgemäß einzusetzenden Perfluoroalkansulfonsäuren gehören u. a. CF₃SO₃H, C₂F₅-SO₃H,
n-C₄F₉-SO₃H, C₆F₁₃-SO₃H, CF₃-CFH-CF₂-SO₃H, 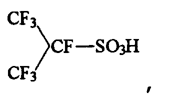 CHF₂-CF₂-SO₃H, C₃F₇-CFH-CF₂-SO₃H, C₅F₁₁-CFH-CF₂-SO₃H, CFCl₂-CF₂₋CFH-CF₂-SO₃H;

Besonders bevorzugt unter den Verbindungen der allgemeinen Formel III sind im Rahmen der Erfindung CF₃SO₃H, CF₃-CFH-CF₂-SO₃H und/oder n-C₄F₉-SO₃H.

Die mit der Erfindung verbundenen Vorteile lassen sich bereits durch einen Zusatz der Polyfluoralkansulfonsäuren der allgemeinen Formel III zu einem ansonsten gebräuchlichen Reaktionsmedium für die Direktfluorierung zumindest teilweise erzielen. Zweckmäßig ist hierbei ein Anteil von mehr als 50 Gew.-% (wt/wt) bezogen auf das gesamte Gewicht des Reaktionsmediums.

Besonders ausgeprägt treten die erfindungsgemäßen Vorteile jedoch hervor, wenn die Direktfluorierung in Polyfluoroalkansulfonsäuren der allgemeinen Formel III als Reaktionsmedium durchgeführt wird. Hierbei sind zwar auch nebengeordnete Mengen anderer Lösungs- oder Verdünnungsmittel nicht ausgeschlossen, deren Zusatz ist jedoch auf geringe Anteile (< 10 Vol-% (v/v)) beschränkt.

In besonders zweckmäßiger Verfahrensvariante werden lediglich Verbindungen der allgemeinen Formel III (entweder rein oder im Gemisch von zwei oder mehreren unter die allgemeine Formel III fallenden Verbindungen) eingesetzt.

In vorteilhafter Abwandlung des erfindungsgemäßen Verfahrens kennzeichnet sich das Verfahren zu Herstellung fluorierter Aromaten weiterhin dadurch, daß die zu fluorierende Verbindung der allgemeinen Formel II mit Polyfluoroalkansulfonsäuren der allgemeinen Formel III als Reaktionsmedium verdünnt oder darin gelöst wird und gasförmiges Fluor in das Reaktionsmedium eingeleitet wird. Dies kann direkt in molekularer Form geschehen, zweckmäßigerweise wird das Fluor zur besseren Kontrolle der Reaktion zusammen mit einem inerten Trägergas durch das Reaktionsmedium geleitet.

Dabei kann das Verhältnis von Fluorgas zu Trägergas je nach angestrebtem Zweck über einen weiten Zusammensetzungsbereich variieren. Vorteilhaft beträgt das Verhältnis von Fluor zu Trägergas zwischen etwa 3 und 25 Volumenprozent (v/v).

Als Trägergas kommen grundsätzlich alle für die Reaktionspartner inerten gasförmigen Stoffe in Frage. Besonders vorteilhaft aufgrund seiner leichten Verfügbarkeit in ausreichender Reinheit ist die Verwendung von Stickstoff als Trägergas.

Das stöchiometrische Verhältnis von Fluor zu Aromaten ist zunächst für die Fluorierung an sich nicht besonders kritisch. Allerdings ist von der Menge an eingesetztem Fluor unter anderem die Art und Zusammensetzung der erhaltenen Produkte abhängig.

In einer Variante ist das Verfahren der Erfindung dadurch gekennzeichnet, daß Verbindungen der allgemeinen Formel II fluoriert werden, worin Z für H steht, und gasförmiges Fluor in einem Verhältnis von 1,1:1 bis 2:1 Mol bezogen auf die vorgelegten Mol der zu fluorierenden aromatischen Verbindung in das Reaktionsmedium eingeleitet wird, so daß bei der eintretenden Reaktion zwischen Aromaten und Fluor die Monofluorierung überwiegt.

In einer alternativen Verfahrensvariante werden Verbindungen der allgemeinen Formel II fluoriert, worin Z für H steht, wobei gasförmiges Fluor in einem Verhältnis von 2,1:1 bis 4:1 Mol bezogen auf die vorgelegten Mol der zu fluorierenden aromatischen Verbindung in das Reaktionsmedium eingeleitet wird, so daß bei der eintretenden Reaktion zwischen Aromaten und Fluor die Difluorierung überwiegt.

Die Temperatur während der Direktfluorierung liegt bevorzugt in einem Bereich von etwa 0 bis 30°C.

Besonders zweckmäßig sind Temperaturen von etwa 10 bis 25°C. Ganz besonders bevorzugt wird die Fluorierung einfach bei Raumtemperatur von etwa 25°C durchgeführt.

Die mit der Erfindung erzielbaren Ausbeuten an Monofluorderivaten betragen zwischen etwa 60 und 90 Gew.-%, insbesondere etwa 80 Gew.-%. Der Umsatz bewegt sich zwischen 80 und 99 %, bezogen auf die in der Reaktion eingesetzte Menge der aromatischen Edukte.

Gegenstand der Erfindung ist auch die Verwendung von Polyfluoroalkansulfonsäuren der allgemeinen Formel III

CFₙH₃₋ₙ(CFY)ₘ-SO₃H (III)

worin
m 0 oder eine positive ganze Zahl aus dem Bereich 1 bis 5, n eine positive ganze Zahl aus dem Bereich 1 bis 3 und Y F, Cl, H oder RFY' ist, wobei Y' F, Cl oder H ist und R C₁₋₃-Alkylen, linear oder verzweigt, ist, welches gegebenenfalls teilweise fluoriert oder auch perfluoriert sein kann,
in einem Reaktionsmedium bei der Direktfluorierung von aromatischen Verbindungen der allgemeinen Formel II 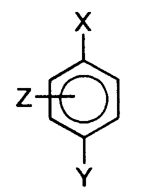 worin X für COOH, COOR, CONH₂, CONR¹R², CF₃, CN, CHO, NO₂ oder NH₂, Y für F, Cl, OCH₃, OCF₃, OCCl₃, p-NO₂-C₆H₄, p-NO₂-C₆H₄-O oder OH, und Z für H, F, Cl, CF₃, CCl₃ oder OCH₃ steht,
wobei R, R¹ und R² unabhängig voneinander gleich oder verschieden C₁-C₆-Alkyl, linear oder verzweigt, sein können und die Alkylreste R, R¹ und R² gegebenenfalls bis zu dreifach mit Halogen substituiert sein können.

Dabei ist ganz besonders bevorzugt, die Verwendung der Polyalkansulfonsäuren der allgemeinen Formel III nicht nur in einem Reaktionsmedium sondern als Reaktionsmedium, was bedeutet ohne Zugabe weiterer Verdünnungs- oder Lösungsmittel.

Die nachfolgenden Beispiele dienen zur Veranschaulichung des Gegenstands der Erfindung.

### Beispiel 1

Eine Lösung von 0,1 mol p-Chlornitrobenzol in 50 ml Polyfluoralkansulfonsäure (CF₃-CFH-CF₂-SO₃H) wurde in einen zur Direktfluorierung geeigneten Reaktor gefüllt. Durch die Lösung wurde während 15 min ein Strom von N₂ geleitet. Anschließend wurde dem Stickstoffstrom eine bestimmte Menge F₂ zugesetzt, so daß eine 10 - 20 Vol-% ige (v/v) Mischung mit dem Stickstoff resultierte. Das Gemisch aus Trägergas und Fluor wurde mit einem Fluß von etwa 10 - 20 ml/min durch die Lösung des Substrats im Reaktionsmedium geführt. Das Gemisch aus Stickstoff und Fluor wurde solange durch das Reaktionsmedium geleitet, bis die durchgeleitete Menge Fluor etwa 0,12 mol entsprach. Anschließend wurde die Reaktionsmischung noch 20 min mit reinem Stickstoff gespült, um nicht verbrauchte Reste des F₂ zu entfernen.

Zur Aufarbeitung wurde die Reaktionsmischung in einen Überschuß Wasser gegossen und die festen Bestandteile wurden abfiltriert, gegebenenfalls zur Gewinnung flüssiger Produkte mit CH₂Cl₂ extrahiert, mit Wasser gewaschen und getrocknet. Die erhaltene Produktmischung kann mit FC, GCMS und ¹⁹F NMR analysiert werden. Die weitere Reinigung der Produkte erfolgt durch Umkristallisation aus geeigneten Lösungsmitteln oder fraktionierte Destillation bei Atmosphärendruck oder unter vermindertem Druck.

Die Gesamtausbeute an aromatischen Produkten betrug 88 Gew.-% (wt/wt).

Die Produktzusammensetzung war:
- 66 Gew.-%: (wt/wt) 3-Fluor-4-chlor-nitrobenzol,
- 33 Gew.-%: (wt/wt) 4-Chlor-nitrobenzol und
- 1 Gew.-%: (wt/wt) 3,5-Difluor-4-chlor-nitrobenzol.

### Beispiel 2

Eine Lösung von 0,1 mol p-Chlornitrobenzol in 50 ml Polyfluoralkansulfonsäure (CF₃-CFH-CF₂-SO₃H) wurde wie in Beispiel 1 beschrieben direkt fluoriert, mit dem Unterschied, daß ein Gemisch aus Stickstoff und Fluor solange durch das Reaktionsmedium geleitet wurde, bis die durchgeleitete Menge Fluor etwa 0,15 mol entsprach.

Zur Aufarbeitung wurde die fertig reagierte Reaktionsmischung in einen Überschuß Wasser gegossen und die festen Bestandteile wurden abfiltriert, gegebenenfalls zur Gewinnung flüssiger Produkte mit CH₂Cl₂ extrahiert, mit Wasser gewaschen und getrocknet. Die erhaltene Produktmischung kann mit GC, GCMS und ¹⁹F NMR analysiert werden. Die weitere Reinigung der Produkte erfolgt durch Umkristallisation aus geeigneten Lösungsmitteln oder fraktionierte Destillation bei Atmosphärendruck oder unter vermindertem Druck.

Die Gesamtausbeute an aromatischen Produkten betrug 91 Gew.-% (wt/wt).

Die Produktzusammensetzung war:
- 78 Gew.-%: (wt/wt) 3-Fluor-4-chlor-nitrobenzol,
- 19 Gew.-%: (wt/wt) 4-Chlor-nitrobenzol und
- 3 Gew.-%: (wt/wt) 3,5-Difluor-4-chlor-nitrobenzol.

### Vergleichsbeispiel 2a

wie Beispiel 2 mit dem Unterschied, daß eine Lösung von 0,05 mol p-Chlornitrobenzol in 50 ml konz. H₂SO₄ anstatt Polyfluoralkansulfonsäure (CF₃-CFH-CF₂-SO₃H) direkt fluoriert wurde.

Die Gesamtausbeute an aromatischen Produkten betrug 75 Gew.-% (wt/wt).

Die Produktzusammensetzung war:
- 63 Gew.-%: (wt/wt) 3-Fluor-4-chlor-nitrobenzol,
- 35 Gew.-%: (wt/wt) 4-Chlor-nitrobenzol und
- 2 Gew.-%: (wt/wt) 3,5-Difluor-4-chlor-nitrobenzol.

### Beispiel 3

Eine Lösung von 0,1 mol p-Chlornitrobenzol in 50 ml Polyfluoralkansulfonsäure wurde wie in Beispiel 1 beschrieben direkt fluoriert, mit dem Unterschied, daß ein Gemisch aus Stickstoff und Fluor solange durch das Reaktionsmedium geleitet wurde, bis die durchgeleitete Menge Fluor etwa 0,2 mol entsprach.

Zur Aufarbeitung wurde die fertig reagierte Reaktionsmischung in einen Überschuß Wasser gegossen und die festen Bestandteile wurden abfiltriert, gegebenenfalls zur Gewinnung flüssiger Produkte mit CH₂Cl₂ extrahiert, mit Wasser gewaschen und getrocknet. Die erhaltene Produktmischung kann mit GC, GCMS und ¹⁹F NMR analysiert werden. Die weitere Reinigung der Produkte erfolgt durch Umkristallisation aus geeigneten Lösungsmitteln oder fraktionierte Destillation bei Atmosphärendruck oder unter vermindertem Druck.

Die Gesamtausbeute an aromatischen Produkten betrug 87 Gew.-% (wt/wt).

Die Produktzusammensetzung war:
- 85 Gew.-%: (wt/wt) 3-Fluor-4-chlor-nitrobenzol,
- 3 Gew.-%: (wt/wt) 4-Chlor-nitrobenzol und
- 12 Gew.-%: (wt/wt) 3,5-Difluor-4-chlor-nitrobenzol.

### Beispiel 4

Eine Lösung von 0,1 mol p-Fluornitrobenzol in 50 ml Polyfluoralkansulfonsäure (n-C₄F₉-SO₃H) wurde wie in Beispiel 1 beschrieben in einen zur Direktfluorierung geeigneten Reaktor gefüllt und fluoriert. Dabei wurde das Gemisch aus Trägergas und Fluor solange durch das Reaktionsmedium geleitet, bis die durchgeleitete Menge Fluor etwa 0,12 mol entsprach.

Zur Aufarbeitung wurde das Produkt unter reduziertem Druck (0,01 mbar) abdestilliert und durch fraktionierte Destillation im Vakuum gereinigt.

Die Gesamtausbeute an aromatischen Produkten betrug 87 Gew.-% (wt/wt).

Die Produktzusammensetzung war:
- 75 Gew.-%: (wt/wt) 3,4-Difluor-nitrobenzol,
- 21 Gew.-%: (wt/wt) 4-Fluor-nitrobenzol und
- 4 Gew.-%: (wt/wt) 3,4,5-Trifluor-nitrobenzol.

### Beispiel 5

Eine Lösung von 0,1 mol p-Fluornitrobenzol in 50 ml Polyfluoralkansulfonsäure (n-C₄F₉-SO₃H) wurde wie in Beispiel 1 beschrieben in einen zur Direktfluorierung geeigneten Reaktor gefüllt und fluoriert. Dabei wurde das Gemisch aus Trägergas und Fluor solange durch das Reaktionsmedium geleitet, bis die durchgeleitete Menge Fluor etwa 0,18 mol entsprach.

Zur Aufarbeitung wurde das Produkt unter reduziertem Druck (0,01 mbar) abdestilliert und durch fraktionierte Destillation im Vakuum gereinigt.

Die Gesamtausbeute an aromatischen Produkten betrug 90 Gew.-% (wt/wt).

Die Produktzusammensetzung war:
- 88 Gew.-%: (wt/wt) 3,4-Difluor-nitrobenzol,
- 2 Gew.-%: (wt/wt) 4-Fluor-nitrobenzol und
- 10 Gew.-%: (wt/wt) 3,4,5-Trifluor-nitrobenzol.

### Vergleichsbeispiel 5a

wie Beispiel 5 mit dem Unterschied, daß eine Lösung von 0,05 mol p-Fluornitrobenzol in 50 ml konz. H₂SO₄ direkt fluoriert wurde.

Die Gesamtausbeute an aromatischen Produkten betrug 76 Gew.-% (wt/wt).

Die Produktzusammensetzung war:
- 71 Gew.-%: (wt/wt) 3,4-Difluor-nitrobenzol,
- 19 Gew.-%: (wt/wt) 4-Fluor-nitrobenzol und
- 10 Gew.-%: (wt/wt) 3,4,5-Trifluor-nitrobenzol.

### Beispiel 6

Eine Lösung von 0,1 mol 2,4-Difluorbenzoesäure in 50 ml Polyfluoralkansulfonsäure (n-C₄F₉-SO₃H) wurde wie in Beispiel 1 beschrieben in einen zur Direktfluorierung geeigneten Reaktor gefüllt und fluoriert. Dabei wurde das Gemisch aus Trägergas und Fluor solange durch das Reaktionsmedium geleitet, bis die durchgeleitete Menge Fluor etwa 0,20 mol entsprach.

Zur Aufarbeitung wurde das Reaktionsmedium unter reduziertem Druck (0,01 mbar) abdestilliert und das zurückbleibende Produkt durch Kristallisation aus Methanol gereinigt.

Die Gesamtausbeute an aromatischen Produkten betrug 91 Gew.-% (wt/wt).

Die Produktzusammensetzung war:
- 75 Gew.-%: (wt/wt) 2,4,5-Trifluorbenzoesäure und
- 25 Gew.-%: (wt/wt) 2,3,4-Trifluorbenzoesäure.

### Beispiel 7

Eine Lösung von 0,1 mol p-Nitro-Trifluoranisol in 50 ml Polyfluoralkansulfonsäure (n-C₄F₉-SO₃H) wurde wie in Beispiel 1 beschrieben in einen zur Direktfluorierung geeigneten Reaktor gefüllt und fluoriert. Dabei wurde das Gemisch aus Trägergas und Fluor solange durch das Reaktionsmedium geleitet, bis die durchgeleitete Menge Fluor etwa 0,18 mol entsprach.

Zur Aufarbeitung wurde das Produkt durch fraktionierte Destillation im Vakuum gereinigt.

Die Gesamtausbeute an aromatischen Produkten betrug 93 Gew.-% (wt/wt).

Die Produktzusammensetzung war:
- 92 Gew.-%: (wt/wt) 2-Fluor-4-nitro-trifluoranisol,
- 6 Gew.-%: (wt/wt) 4-Nitro-Trifluoranisol und
- 2 Gew.-%: (wt/wt) 2,5-Difluor-4-nitro-trifluoranisol

### Beispiel 8

Eine Lösung von 0,1 mol p-Nitro-phenol in 50 ml Polyfluoralkansulfonsäure (CF₃-SO₃H) wurde wie in Beispiel 1 beschrieben in einen zur Direktfluorierung geeigneten Reaktor gefüllt und fluoriert. Dabei wurde das Gemisch aus Trägergas und Fluor solange durch das Reaktionsmedium geleitet, bis die durchgeleitete Menge Fluor etwa 0,12 mol entsprach.

Zur Aufarbeitung wurde das Reaktionsmedium unter reduziertem Druck (0,1 mbar) abdestilliert und das zurückbleibende Produkt durch Kristallisation gereinigt.

Die Gesamtausbeute an aromatischen Produkten betrug 89 Gew.-% (wt/wt).

Die Produktzusammensetzung war:
- 94 Gew.-%: (wt/wt) 2-Fluor-4-nitro-phenol,
- 2 Gew.-%: (wt/wt) 4-Nitro-phenol und
- 4 Gew.-%: (wt/wt) 2,5-Difluor-4-nitro-phenol.

Die nachfolgende Tabelle gibt einen Überblick über die in den Beispielen eingesetzten Substrate, die zur Direktfluorierung verwendete Menge Fluor, die Ausbeute an Gesamtprodukt sowie die Zusammensetzung der jeweiligen erhaltenen aromatischen Produkte.

Weitere Vorteile und Ausführungsformen der Erfindung ergeben sich aus den nachfolgenden Patentansprüchen.

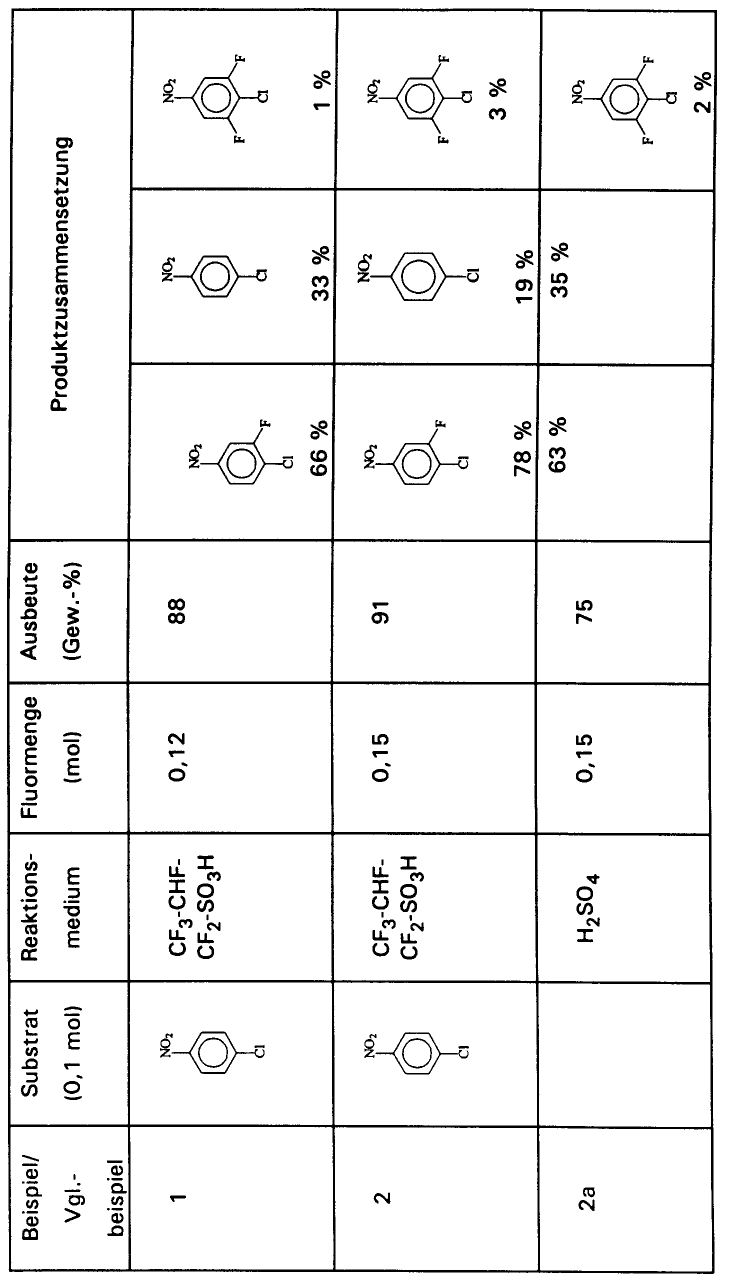

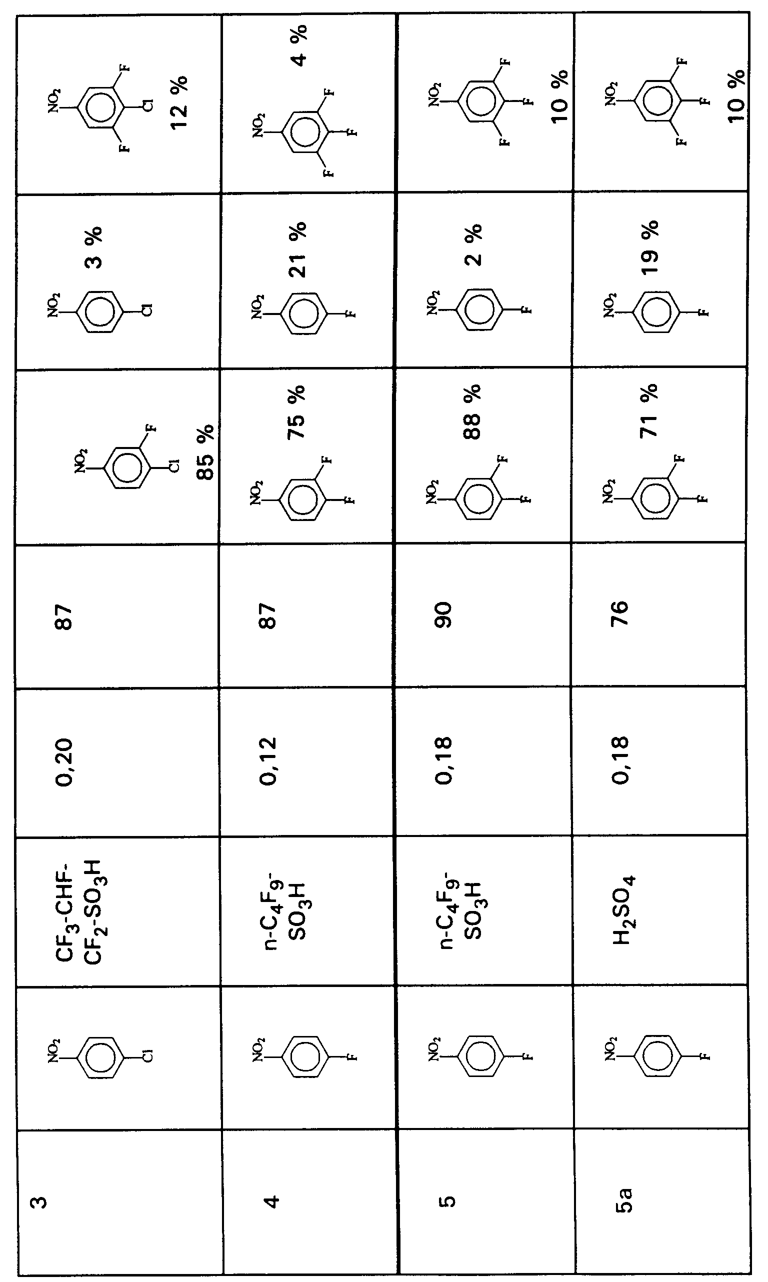

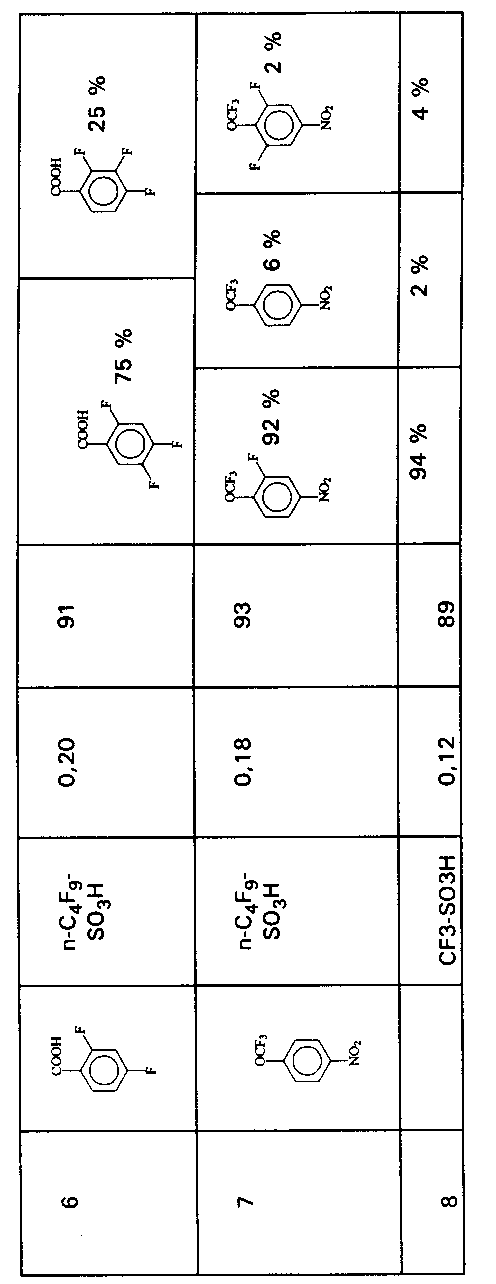

### Beispiel 9

Eine Lösung von 0,1 mol p-Fluornitrobenzol in 50 ml Trifluoromethansulphonsäure (CF₃-SO₃H) wurde wie in Beispiel 1 beschreiben direkt fluoriert, mit dem Unterschied, daß ein Gemisch aus Stickstoff und Fluor solange durch das Reaktionmedium geleitet wurde, bis die durchgeleitete Menge von Fluor etwa 0,3 mol entsprach. Zur Aufarbeitung wurde die fertig reagierte Reaktionmischung in einen Überschuß Wasser gegossen und die Produkte mit CH₂Cl₂ extrahiert, mit Wasser gewaschen und getrocknet. Die erhaltene Produktmischung wurde mit GC und 19F NMR analysiert. Die weitere Reinigung der Produkte erfolgt durch fraktionierte Destillation unter verminderten Druck.
Die Gesamtausbeute an aromatischen Produkten betrug 63 Gew.-% (wt/wt).
Die Produktzusammensetzung war:
- 3 Gew.-%: (wt/wt) 4-Fluornitrobenzol
- 20 Gew.-%: (wt/wt) 3,4-Difluornitrobenzol
- 70 Gew.-%: (wt/wt) 3,4,5-Trifluornitrobenzol
- 7 Gew.-%: (wt/wt) 1,4,5-Trifluornitrobenzol

## Patentansprüche

1. Verfahren zur Herstellung von fluorierten aromatischen Verbindungen der allgemeinen Formel I 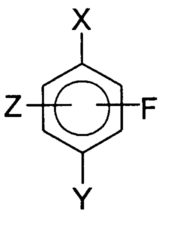 worin
X für COOH, COOR, CONH₂, CONR¹R², CF₃, CN, CHO, NO₂ oder NH₂,
Y für F, Cl, OCH₃, OCF₃, OCCl₃, p-NO₂-C₆H₄, p-NO₂-C₆H₄-O oder OH, und Z für H, F, Cl, CF₃, CCl₃ oder OCH₃ steht,
wobei R, R¹ und R² unabhängig voneinander gleich oder verschieden C₁-C₆-Alkyl, linear oder verzweigt, sein können und die Alkylreste R, R¹ und R² gegebenenfalls bis zu dreifach mit Halogen substituiert sein können,
durch Umsetzung von aromatischen Verbindungen der allgemeinen Formel II 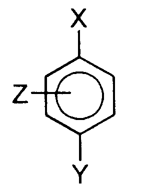 worin
X, Y und Z die bei Formel I angegebene Bedeutung besitzen,
mit Fluor in einem Reaktionsmedium dadurch gekennzeichnet,
daß die Direktfluorierung in einem Reaktionsmedium enthaltend Polyfluoroalkansulfonsäuren der allgemeinen Formel III
CFnH₃₋ₙ(CFY)ₘ-SO₃H (III)
worin
m 0 oder eine positive ganze Zahl aus dem Bereich 1 bis 5, n eine positive ganze Zahl aus dem Bereich 1 bis 3 und Y F, Cl, H oder RFY' ist, wobei Y' F, Cl oder H ist und R C₁₋₃-Alkylen, linear oder verzweigt, ist, welches gegebenenfalls teilweise fluoriert oder auch perfluoriert sein kann, durchgeführt wird.

2. Verfahren nach Anspruch 1, dadurch gekennzeichnet, daß die Direktfluorierung in Polyfluoroalkansulfonsäuren der allgemeinen Formel III als Reaktionsmedium durchgeführt wird.

3. Verfahren nach Anspruch 1 oder 2, dadurch gekennzeichnet, daß die zu fluorierende Verbindung der allgemeinen Formel II mit Polyfluoroalkansulfonsäuren der allgemeinen Formel III als Reaktionsmedium verdünnt oder darin gelöst wird und gasförmiges Fluor in das Reaktionsmedium eingeleitet wird.

4. Verfahren nach Anspruch 3, dadurch gekennzeichnet, daß das Fluor zusammen mit einem inerten Trägergas durch das Reaktionsmedium geleitet wird.

5. Verfahren nach Anspruch 4, dadurch gekennzeichnet, daß das Verhältnis von Fluor zu Trägergas zwischen etwa 3 und 25 Volumenprozent (v/v) beträgt.

6. Verfahren nach Anspruch 5, dadurch gekennzeichnet, daß als Trägergas Stickstoff verwendet wird.

7. Verfahren nach einem oder mehreren der vorhergehenden Ansprüche, dadurch gekennzeichnet, daß CF₃SO₃H, CF₃-CFH-CF₂-SO₃H und/oder n-C₄F₉-SO₃H als Reaktionsmedium verwendet wird.

8. Verfahren nach einem oder mehreren der vorhergehenden Ansprüche, dadurch gekennzeichnet, daß Verbindungen der allgemeinen Formel II fluoriert werden, worin Z für H steht, und gasförmiges Fluor in einem Verhältnis von 1,1:1 bis 2:1 Mol bezogen auf die vorgelegten Mol der zu fluorierenden aromatischen Verbindung in das Reaktionsmedium eingeleitet wird, so daß bei der eintretenden Reaktion zwischen Aromaten und Fluor die Monofluorierung überwiegt.

9. Verfahren nach einem oder mehreren der vorhergehenden Ansprüche 1 bis 7, dadurch gekennzeichnet, daß Verbindungen der allgemeinen Formel II fluoriert werden, worin Z für H steht, und gasförmiges Fluor in einem Verhältnis von 2,1:1 bis 4:1 Mol bezogen auf die vorgelegten Mol der zu fluorierenden aromatischen Verbindung in das Reaktionsmedium eingeleitet wird, so daß bei der eintretenden Reaktion zwischen Aromaten und Fluor die Difluorierung überwiegt.

10. Verfahren nach einem oder mehreren der vorhergehenden Ansprüche, dadurch gekennzeichnet, daß die Direktfluorierung bei Temperaturen zwischen etwa 0 und 30°C durchgeführt wird.

11. Verfahren nach Anspruch 10, dadurch gekennzeichnet, daß die Fluorierung bei Raumtemperatur von etwa 25°C durchgeführt wird.

12. Verwendung von Polyfluoroalkansulfonsäuren der allgemeinen Formel III
CFₙH₃₋ₙ(CFY)ₘ-SO₃H (III)
worin
m 0 oder eine positive ganze Zahl aus dem Bereich 1 bis 5, n eine positive ganze Zahl aus dem Bereich 1 bis 3 und Y F, Cl, H oder RFY' ist, wobei Y' F, Cl oder H ist und R C₁₋₃-Alkylen, linear oder verzweigt, ist, welches gegebenenfalls teilweise fluoriert oder auch perfluoriert sein kann,
in einem Reaktionsmedium bei der Direktfluorierung von aromatischen Verbindungen der allgemeinen Formel II 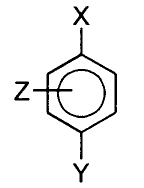 worin
X für COOH, COOR, CONH₂, CONR¹R², CF₃, CN, CHO, NO₂ oder NH₂,
Y für F, Cl, OCH₃, OCF₃, OCCl₃, p-NO₂-C₆H₄, p-NO₂-C₆H₄-O oder OH, und Z für H, F, Cl, CF₃, CCl₃ oder OCH₃ steht,
wobei R, R¹ und R² unabhängig voneinander gleich oder verschieden C₁-C₆-Alkyl, linear oder verzweigt, sein können und die Alkylreste R, R¹ und R² gegebenenfalls bis zu dreifach mit Halogen substituiert sein können.

13. Verwendung nach Anspruch 12 als Reaktionsmedium.
